# EUROPEAN PATENT SPECIFICATION

(11) **EP 0 152 820 B1**
(45) Date of publication and mention of the grant of the patent: **17.04.1996**
(21) Application number: 85100901.9
(22) Date of filing: 29.01.1985
(51) Int. Cl.: C07C 271/08, C08G 71/00, C09D 5/44

(54) **Carbamate compounds, compositions and method of making the same**
Carbamidsäureester-Derivate, Mittel und Herstellungsverfahren
Dérivés à fonction carbamate, compositions et méthodes de préparation

(30) Priority: 17.02.1984 US 581006; 17.02.1984 US 581015; 17.02.1984 US 581011; 17.02.1984 US 581007; 17.02.1984 US 581009; 17.02.1984 US 581005; 17.02.1984 US 581010; 17.02.1984 US 581012; 17.02.1984 US 581008; 17.02.1984 US 581013
(43) Date of publication of application: 28.08.1985
(60) Divisional application: 95110663.2
(73) Proprietor: CYTEC TECHNOLOGY CORP., Wilmington, Delaware 19801 (US)
(72) Inventor: Parekh, Girish Girdhar, Fairfield Connecticut 06432 (US); Jacobs III, William, Bridgeport Connecticut 06605 (US); Blank, Werner Josef, Wilton Connecticut 06894 (US)
(74) Representative: Diehl, Hermann O. Th., Dr.

(56) References cited:
- EP-A- 0 121 837
- DE-A- 3 246 812
- DE-C- 1 248 364

## Description

The present invention concerns a thermosettable coating composition and a method of forming a coating on a substrate.

The reaction of propylene carbonate with primary and secondary amines to produce corresponding 2-hydroxypropyl carbamates is known in the art (Comp. rend, 1142, 1954). Similar reactions of ethylene carbonate are exemplified by the article, "The Preparation of Polymeric and Cyclic Urethans and Ureas from Ethylene Carbonate and Amines" by Elizabeth Dyer and Harvey Scott, J.A.C.S. (1956) pp. 672 - 675. See also the report "Polyurethane elastomers obtained without the use of diioscyanates" by L. Ya. Rappoport, G.N. Petrov, I.I. Trostyanskaya and O.P.D. Gavrilova in International Polymer Science and Technology, 8, No. 1, 1981. The Dyer-Scott reference discloses that polyurethanes might be prepared from 2-(hydroxyethyl)- carbamate by elimination of ethylene glycol, thereby avoiding the need for using diisocyanates. The Rappoport et al paper discloses generally the reaction of cyclic carbonates with amines to form polyurethane elastomers. Thus, the prior art shows an awareness that amines react with, e.g., propylene carbonate, to yield the corresponding hydroxyalkyl carbamates. The Journel of Polymer Science, Vol. 7, 899 916 (1969), in an article entitled "New Method for Preparing Saturated and Unsaturated Aliphatic Polyurethanes" by Y. Mizake, S. Ozaki and Y. Hirata, at pages 899 - 915, discloses alternate routes to saturated and unsaturated polyurethanes, including polycondensation reaction of glycol bis(chloroformate) with diamine.

An article by Richard D. Cowell entitled: "Thermoplastic Polyurethane Elastosmers: Chemistry Properties and Processing for the 80's in the Journal of Elastomers and Plastics, Vol. 14, (October, 1982) pages 195 - 203, discloses the preparation of bis(2-hydroxyethyl) carbamates by reaction of diamines with ethylene carbonate followed by a catalyzed transesterification reaction with a glycol or macroglycol. Two types of diamines were used, 1, 4 cyclohexane - bis- (methyl-amine) and a product sold under the trademark Jeffamine D2000 comprising a diamine with a polyoxypropylene backbone.

Coating compositions comprising a cross-linker and a backbone polymer containing sites thereon which are reactive with the cross-linker at elevated temperature to form a cross-linked polymeric material, but which are stable relative to each other at ambient temperatures are of course well known in the art. One difficulty with such compositions is that the high viscosity and high softening temperature of backbone polymers typically employed requires the utilization of a solvent to reduce viscosity of the polymer. After application of the coating composition and heating it to cure, volatilization of the solvent produces environmental, health and processing problems.

In order to achieve high solids content coatings compositions, it is known to use high boiling point diols or polyols as reactive diluents in the compositions. However, in acid-catalyzed paint or coating formulations containing amino cross-linkers, the presence of diols or polyols reduces the shelf life of the coating formulation because the hydroxy groups on the polymer react with the amino cross-linkers.

The use of blocked or capped isocyanates as cross-linking agents which are stable or nonreactive with functional groups such as hydroxyl or amine groups at room temperature, but which react to cross-link therewith at elevated temperature, is known in the art. For example, U.S. Patent 3,984,299 of Robert D. Jerabek discloses an electrodepositable composition comprising an amine adduct of an epoxy group containing resin, a capped or blocked organic polyisocyanate and, optionally, a catalyst for urethane formation. The blocked isocyanate is disclosed as being any isocyanate in which the isocyanate groups have been reacted with a compound such as an aliphatic, cycloaliphatic, aromatic alkyl monoalcohol or phenolic compound.

The art shows generally the use of blocked isocyanates as cross-linking agents in coating applications such as powder coatings, electrodepositable coatings, and aqueous and organic solvent-borne coatings. Blocked isocyanates are usually prepared by reacting a blocking agent, such as monoalcohols, phenols, lactams, oximes, beta-dicarbonyl compounds, triazoles, hydroxamic acid esters and the like, with aliphatic or aromatic polyisocyanates. See, for example, Progress in Organic Coatings Review, 9, 1981 Z. Wicks.

One disadvantage associated with the utilization of blocked isocyanate compounds is the necessity of storing and handling these toxic compounds. Another difficulty is the relatively high cost of suitable isocyanate compounds as compared to the compounds utilized in the present invention.

Electrodepositable resin compositions are of course well known in the art. For example, U.S. Patent 4,031,050 discloses cationic electrodepositable compositions of blocked organic polyisocyanates and an amine adduct of an epoxy resin. As disclosed in this patent, electrodeposition of such compounds, which may optionally contain a catalyst for urethane formation, can be carried out to provide coatings on a conductive substrate, which coatings have desirable properties. In this regard, see also U.S. Patents 3,984,299 and 4,031,050. However, isocyanate compounds are toxic and highly reactive, requiring the taking of suitable precautions in handling and storing the same.

U.S. Patent 4,017,438 discloses an epoxy resin- derived, cationic electrodepositable resin enhanced by the incorporation of primary amine groups into the resin molecule, by reacting certain polyamine compounds having primary amine groups blocked by ketimine. The ketimine groups when contacted with water, will decompose to provide primary amine functionality as disclosed in this patent. Capped isocyanates are disclosed in combination with the amine-resin adduct to provide, together with a suitable catalyst a cationically electrodepositable resin system. The electrodeposited coating, upon being heated to an elevated temperature, usually in the presence of a cross-linking catalyst, undergoes cross-linking through urethane, hydroxy and amino groups.

As well known, the "capped" or "blocked" isocyanates react with hydroxyl groups and amino groups under conditions of elevated temperature to form urethane and urea cross linkages.

Coating systems based on organic solvent-based materials, such as isocyanate systems, are available which provide high performance, urethane cross-linked coatings but engender enviornmental and fire hazards because of the use of volatile or toxic organic solvents. Commercially available isocyanate compounds typically are toxic and highly reactive, requiring the taking of suitable precautions in handling and storing the same. Aqueous solutions or dispersions of polyurethanes for coatings are known; but these known systems usually require high curing temperatures on the order of 350 to 600°F(176 to 315° C) in order to obtain cross-linking through the urethane groups. Although other low temperature-, or even room temperature-curable aqueous solutions or dispersions of isocyanate-free polyurethanes are available, such coatings do not cross-link through urethane groups and therefore are not likely to meet the performance standards attainable by urethane cross-linked coatings. Most aqueous dispersions of polyurethanes are usually attained by the addition of acids to form cationic dispersions or by the addition of bases to form anionic dispersions, or by the addition of surfactants, all of which additives can adversely affect the properties of the cured film obtained thereby. For example, the aqueous cationic-, anionic-, or surfactant-dispersed isocyanate-based polyurethanes often suffer from a lack of stability upon aging. If there are -NCO groups present, a reaction between water and the isocyanate will usually take place within about three to twenty hours at room temperature. Thus, isocyanate-based polyelectrolytes which are fully soluble in water either readily hydrolyze in water or, after removal of water, become brittle and ' hygroscopic. Because of these drawbacks occasioned by the high ion group content of such materials, they are not of significant practical importance in the field of coatings and plastics generally.

One class of non-ionic aqueous solutions of polyurethanes is based upon the incorporation of polyester-glycol or polyether-glycol segments. However, the polyester-glycol types are sensitive to hydrolytic degradation while the water solubility of the polyether-glycol based resins is isocyanate-dependent. Moreover, both types tend to yield cured films with excessive sensitivity to water, i.e., films which are subject to swelling, turbidity (turning white), softening, and variable adhesion upon exposure to water.

It has now been found that multi-functional amines containing at least one primary and at least one hindered secondary amine group are, unexpectedly, selectively reactive with cyclic carbonates at the primary amine groups, leaving one or more secondary amine groups unreacted in the resulting hydroxyalkyl carbamate. The resultant hydroxyalkyl carbamate contains at least one unreacted secondary amine group and is a useful intermediate in the preparation of polymers which are cross-linkable through hydroxyalkyl carbamate groups.

According to the present invention a thermosettable coating composition is provided comprising:
(a) a polymer selected from the class consisting of one or one or more of hydroxy group-containing polyurethane, hydroxy group-containing polyurea and hydroxy group-containing polyurethane/polyurea polymers;
(b) an amino cross-linker, which is selected from the class consisting of one or more of urea-formaldehyde, melamine formaldehyde, glycoluril-formaldehyde and benzoguanamine formaldehyde resins which have been at least partially alkylated and at least partially methylolated; and
(c) optionally, an acid catalyst; wherein the polymer (a) is the reaction product of one or more reaction routes selected from the class consisting of (i) self-condensation of a poly-hydroxyalkyl carbamate compound, (ii) condensation of a poly-hydroxyalkyl carbamate compound with a polyol, and (iii) condensation of poly-hydroxyalkyl carbamate compound with a polyamine, said poly-hydroxyalkyl carbamate compound having the formula: 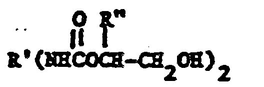 wherein each of R' and R" is the same or different and comprises a suitable organic moiety.

Further, the present invention provides a method of forming a coating on a substrate comprising
(a) applying to the substrate a coating composition comprising:
   (1) a polymer selected from the class consisting of one or more of hydroxy group-containing polyurethane, hydroxy group-containing polyurea and hydroxy group-containing polyurethane/polyurea polymers;
   (2) an amino cross-linker, which is selected from the class consisting of one or more of urea-formaldehyde, melamine formaldehyde, glycoluril-formaldehyde and benzoguanamine formaldehyde resins which have been at least partially alkylated and at least partially methylolated; and
   (3) optionally, an acid catalyst; and
   (4) optionally, a solvent; and
(b) heating the applied composition at an elevated temperature and for a time sufficient to cure it;
   wherein the polymer (1) is the reaction product of one or more reaction routes selected from the class consisting of (i) self-condensation of a poly-hydroxyalkyl carbamate compound, (ii) condensation of a poly-hydroxyalkyl carbamate compound with a polyol, and (iii) condensation of a poly-hydroxyalkyl carbamate compound with a polyamine, said poly-hydroxyalkyl carbamate compound having the formula: 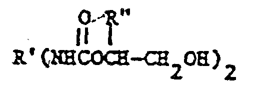 wherein each of R' and R" is the same or different and comprises a suitable organic moiety.

Generally, the poly-hydroxyalkyl carbamate compounds referred to above are made by reacting one or more amines and cyclic carbamates to yield compounds having the above formula

The cyclic carbonate to be reacted with the multi-functional amine may comprise any suitable cyclic carbonate, including biscarbonates, which is reactive with the primary amine groups of a multi-functional amine. Generally, five-member ring organic carbonates are preferred as compared to six-member ring organic carbonates, the latter being relatively more expensive and difficult to prepare. Accordingly, a preferred utilizable cyclic carbonate has the formula: 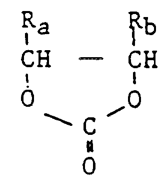 wherein Rₐ and R_{b} may be the same or different, and each may comprise H, or a C₁ to C₈ aliphatic, cycloaliphatic, aromatic or heterocyclic compound. Ethylene carbonate (dioxolane-2-one), both Rₐ and R_{b} = H, and propylene carbonate (4-methyldioxolane-2-one); Rₐ = H and R_{b} = CH₃, are preferred reactants.

The formulas of suitable classes of amines may be derived by simply replacing with -NH₂ the hydroxyalkyl carbamate moieties, of the polyhydroxyalkyl carbamate compounds in accordance with the above general formula.

It will be appreciated by those skilled in the art that a wide variety of aliphatic amines may be used in the invention.

Aliphatic and cycloaliphatic amines react readily with cyclic carbonates to provide the hydroxyalkyl carbamate compounds used in the invention.

Aromatic amines usually require an appropriate catalyst to promote the reaction with cyclic carbonates as shown, for example, in U.S. Patent 4,268,684 of Arthur E. Gurgiolo.

The hydroxy-group-containing polyurethane polymers used in the thermosettable coating composition according to the invention may be prepared either by self-condensing the poly-hydroxyalkyl carbamate compound or by condensing it with a polyol. The polyol employed in the latter condensation reaction may be any suitable polyol and may be derived from polyols, polyether polyols, polyester polyols, alkyd polyols, phenol formaldehyde condensation products, bisphenol-A, bisphenol-F, polyether resins, bisphenol ethylene oxide or propylene oxide condensation products, polyether resins, dihydroxy polybutadiene, or the like.

Typical polyether diols or polyols which are suitable for use in the present invention are polyethers derived from the reaction of diols, polyols or amines with ethylene oxide, propylene oxide, styrene oxide or the like, or mixtures of other epoxies with a carbon content from C₂ to about C₁₈ or polyethers derived from tetrahydrofuran.

Typical polyester diols or polyols which are suitable for use in the present invention can be prepared by known methods of condensing diol, triols or polyols with a combination of mono-, di-, tri-, or polybasic carboxylic acids. Typical diols or polyols are ethylene glycol, propylene glycol, diethylene glycol, dipropylene glycol, 1,3 butane diol, 1,6-hexanediol, neopentyl glycol, cyclohexane- dimethanol, trimethyl pentane diol, trimethylol propane, pentaerythritol, etc. Typical carboxylic acids are C₈ to C₁₈ fatty acids, dimeric fatty acids, C₇ to C₁₀ aliphatic dibasic acids, aromatic mono carboxylic and polycarboxylic acid such as benzoic, phthalic and trimellitic acid.

Polyols or a combination of polyols with an average functionality of 2 to 3 are preferred. Although higher functional polyols can be reacted with the poly-hydroxyalkyl carbamate compounds, the ratio of polyol to carbamate and the resultant reaction must be controlled to prevent gellation. It will be appreciated that one skilled in the art can readily determine, by conventional techniques, the selection of appropriate reactants and conditions so as to avoid gellation. The condensation of the poly-hydroxyalkyl carbamate compound and a polyol may be represented by the following equation. 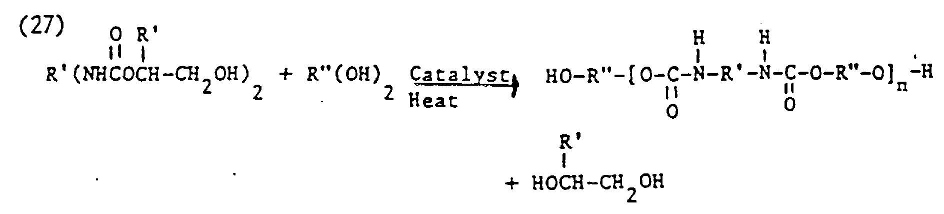 wherein each of R' and R" is the same or different and comprises any suitable organic moiteies.

Reaction (27) illustrates the formation of an hydroxy group-terminated polyurethane polymer and a 1, 2-diol leaving group. The reaction of the poly-hydroxyalkyl carbamate and a polyol can proceed solely by heat or in the presence of a suitable transeterification catalyst such as a tin compound. The reaction can also be run in a solvent medium. The diol which is eliminated during the condensation reaction may be conti-nuously removed by distillation, either using the vacuum or azerotropic distillation with a suitable solvent.

An hydroxy group-containing polyurethane polymer having a high urethane content may be obtained by self-condensation of a poly-hydroxyalkyl carbamate compound as illustrated by 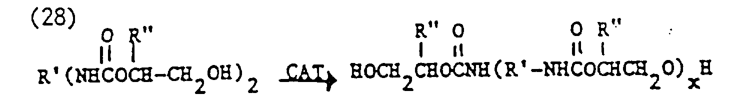 wherein each of R' and R" is the same or different and comprises a suitable organic moiety. The reaction of the poly-hydroxyalkyl carbamate and a polyamine can proceed in the presence of any suitable catalyst. Suitable catalysts include organo tin compounds such as dialkyltin compounds, e.g., dibutyltindilaurate, organo zinc compounds such as zinc octoate, zinc butyrate, etc., some organo titanium compounds, and, generally, any suitable catalysts as are known in the art.

The hydroxy group-containing polyurea polymer may be obtained by condensation of the poly-hydroxyalkyl carbamate compound with a suitable polyamine, as illustrated by 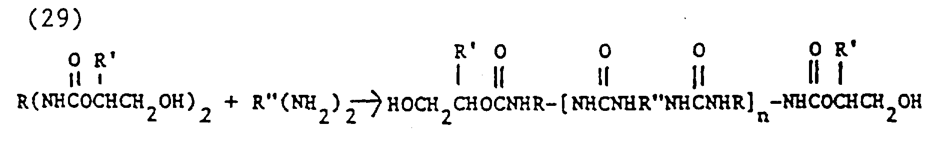 wherein each of R' and R" may be the same or different and comprise any suitable organic moiety.

It should be noted that partial replacement of the polyol with polyamines can be carried out, i.e., a co-condensation of the poly-hydroxyalkyl carbamate compound with one or more polyols and one or more polyamines can be carried out in order to incorporate both urea and urethane linkages in the polymer. For example, the incorporation of urea linkages in the polyurethane polymer is often desired to achieve improved hardness and increased solvent resistance.

While the above-described polyurea and polyurethane polymers may find use as thermoplastics in elastomer applications such as molding compounds, coatings for metals, plastics, textiles or adhesives, it has been recognized that their hydroxy functionality and the urethane linkage or unsaturation on the main chain can be used for further cross-linking. The hydroxy functionality can be cross-linked with amino formaldehyde resins or isocyanates and the unsaturated linkages can be used for air oxidative cure or for sulfur vulcanization. It has been found that a composition well suited to provide a thermosettable coating composition can be prepared by utilizing an amino cross-linker in combination with the polyurethane, polyurea or polyurethane-polyurea polymer.

The amino cross-linker may be any one of a large variety of known amino cross-linking compounds including cross-linkers based on melamine, glycoluril, guanamines such as benzoguanamine, urea, substituted ureas and the like. As used herein, the term "amino cross-linker" is intended to include any suitable aminoplast. One such suitable class of materials is aminoplast resinous' compositions, in particular, modified aminoplast resinous compositions such as those disclosed in U.S. Patent 3,082,180, comprising modified amino-triazine-aldehyde resins. Another suitable class of amino cross-linkers useable as a component of the invention comprises fully methylated, fully metbylolated melamine compositions, a process for'the manufacture of which is described in U.S. Patent 4,293,692. Another suitable group of amino cross-linkers is that described in U.S. Patent 4,105,708 and comprising substantially fully mixed-alkylated, substantially fully methylolated glycoluril derivatives comprising dimethoxymethyl diethoxymethyl glycoluril. Glycoluril is also known as acetylene urea and is obtained by reacting two moles of urea with one of glyoxal. Its proper chemical name is tetrahydroimidazo - (4, 5-d) imidazole 2, (1H, 3H)- dione. The disclosures ot the aforesaid U.S. Patents 3,082,180, 4,293,692 and 4,105,708 are each incorporated by reference herein.

Particularly suitable classes of amino cross-linkers are melamine-formaldehyde resins and glycoluril-formaldehyde resins which, in each case, have been partially or fully, i.e., at least partially, alkylated and methylolated. For example, as disclosed in the aforesaid U.S. Patent 4,293,692, melamine may be methylolated by reaction with formaldehyde. The melamine may be either fully methylolated to produce hexamethylol melamine, or partially methylolated to produce pentamethylol melamine, tetramethylol melamine, etc., or mixtures of two or more of the foregoing. The at least partially methylolated melamine (or glycoluril) may then be reacted with an alcohol, such as methanol, to fully or partially alkylate the fully or partially methylolated melamine or glycoluril. For example, a substantially fully methylolated, fully alkylated melamine (hexamethoxymethyl-melamine) is sold under the trademark CYMEL 303 by American Cyanamid Company. Reference herein and in the claims to "melamine-formaldehyde" and "glycoluril-formaldehyde" resins includes any suitable melamine and any suitable glycoluril derived resin utilizable as a cross-linker in coating compositions. Similarly, reference herein and in the claims to "urea-formaldehyde" and "benzoguanamine-formaldehyde" resins includes corresponding urea and benzoguanamine derived resins.

Any suitable solvent or vehicle may be used to carry the polymer, cross-linker and optional catalyst such as, for example, ethylene glycol monethyl ether (Cellosolve).

The catalyst optionally employed in the composition may comprise any suitable acid catalyst. Generally, any substance meeting the definition of a Lewis or Bronsted acid and which does not interfere with the cross-linking reaction may be used. Among suitable acid catalysts are aromatic sulfonic acid compounds such as those disclosed in U.S. Patent 3,960,688, alkyl esters of phosphoric or alkyl phosphonic acids, dinonyl naphthalene sulfonic acid, paratoluene sulfonic acid, n-dodecylbenzenesulfonic acid and the like, as well as inorganic acids such as nitric, sulfuric, phosphoric and hydrohalic acids. Phosphoric acid ester catalysts of the type sold under the trademark CYCAT by American Cyanamid Company may suitably be employed.

Other additives, such as pigments, modifiers, etc. may also be included in the composition as is well known in the art.

Depending on the application process, either a solid powder or a liquid is applied onto the substrate to be coated and after evaporation of any solvent present, the system is cured for a sufficient period of time, e.g., from several minutes to several hours, at temperatures sufficient to affect cure, e.g., from about 200 to about 400°F (about 93 to 204°C).

The compositions of the present invention are stable at ambient temperature and must be heated to an elevated temperature in order to cause the cross-linking reaction to occur at an appreciable rate. Generally, an elevated temperature of about 200°F-400°F (about 93°C-209°C) or more is required to effectuate the cross-linking reaction at an appreciable rate. As used herein and in the claims, an "elevated" temperature is one which is sufficient to cure the deposited composition by causing the cross-linking reaction to occur at a desired rate, usually a rate sufficient to effectuate cure within a period of 1 to 3 hours or less

### EXAMPLE 1

A polyether polyurethane diol is prepared by adding to a suitably equipped flask 32 grams (0.10 M) of polytetramethylene glycol (sold under the trademark Teracol 650); 78 grams (0.12 M) of hexamethylene bis(hydroxypropyl) carbamate, prepared following a literature procedure (Bull. Chim. Soc. Fr. 1142, 1954); and 0.6 grams dibutyltindilaurate. The reaction mixture was heated to 175°C under reduced pressure.

A distillate carried over under reduced pressure was mainly propylene glycol and a small amount of propylene carbonate. About 12 grams of distillate was collected before terminating the reaction after 2 hours. On cooling, 75 grams of Cellosolve was added to the resulting product which by i.r. showed urethane linkages and very few urea linkages. The final resin solids was 57 ± 2% by weight and the resin displayed a very high viscosity.

### EXAMPLE 2

A polyester polyurethane diol was prepared following the procedure of Example 23, except that 53 grams (0.1M) PCP polyol 0200 was used in place of the Teracol 650 polytetramethylene glycol. The i.r. of the product showed the presence of ester and urethane linkages. No urea or amide links were present. The final resinous product was diluted with 25 grams of Cellosolve. The final resin solids and Gardner-Holdt viscosity were 51 ± 2% by weight and A-B, respectively.

### EXAMPLES 3-8

The urethane group-containing resins of Examples 3-8 below were formulated with a methylated melamine-formaldehyde resin in the presence of a phosphoric acid ester based catalyst to prepare coating compositions as set forth in the Table below.

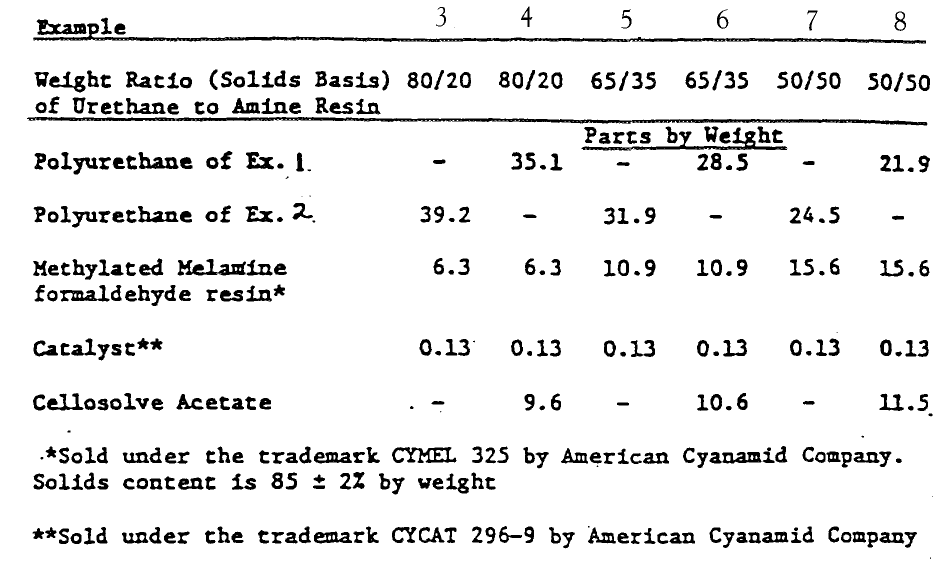

Draw-downs of the above compositions were made on bonderite 100 supports, using a No. 46 wirecater, held-out 10 minutes. The thus applied coatings were cured at 125°C for 20 minutes. The characteristics of the cured coatings thus obtained are shown in the following Table.

**Table**

| Cured Composition of Example: | 3 | 4 | 5 | 6 | 7 | 8 |
|---|---|---|---|---|---|---|
| Film Thickness µm (Mils) | 25.4(1.0) | 25.4(1.0) | 25.4(1.0) | 25.4(1.0) | 25.4(1.0) | 25.4(1.0) |
| Knoop Hardness | - | - | 3.0 | 3.6 | 5.5 | 8.2 |
| Pencil Hardness | 4B | 4B | F-H | HB-F | H-2H | 2H-3H |
| Impact cm/454 g (in/lbs), direct | - | - | 203.2(80) | 254(100) | 25.4(10) | 25.4(10) |
| MEK rubs | 120 | 100 | 200+ | 200+ | 200+ | 200+ |

As indicated by the above Table, the formulations containing urethane/amine resins in a weight ratio of 65/35 (Examples 5 and 6) gave optimum film properties after curing for these specific formulations. All the films had good MEK rub resistance and good flexibility.

## Claims

1. A thermosettable coating composition comprising:
(a) a polymer selected from the class consisting of one or one or more of hydroxy group-containing polyurethane, hydroxy group-containing polyurea and hydroxy group-containing polyurethane/polyurea polymers;
(b) an amino cross-linker, which is selected from the class consisting of one or more of urea-formaldehyde, melamine formaldehyde, glycoluril-formaldehyde and benzoguanamine formaldehyde resins which have been at least partially alkylated and at least partially methylolated; and
(c) optionally, an acid catalyst; wherein the polymer (a) is the reaction product of one or more reaction routes selected from the class consisting of (i) self-condensation of a poly-hydroxyalkyl carbamate compound, (ii) condensation of a poly-hydroxyalkyl carbamate compound with a polyol, and (iii) condensation of poly-hydroxyalkyl carbamate compound with a polyamine, said poly-hydroxyalkyl carbamate compound having the formula: 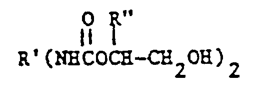 wherein each of R' and R" is the same or different and comprises a suitable organic moiety.

2. The composition of Claim 1 further including (d) a solvent which is compatible with components (a) - (c).

3. The composition of Claim 1 wherein the polyhydroxyalkyl carbamate is hexamethylene bis(hydroxypropyl) carbamate.

4. The composition of Claim 1 wherein the acid catalyst (c) is selected from the class consisting of one or more of Lewis or Bronsted acids.

5. A method of forming a coating on a substrate comprising
(a) applying to the substrate a coating composition comprising:
(1) a polymer selected from the class consisting of one or more of hydroxy group-containing polyurethane, hydroxy group-containing polyurea and hydroxy group-containing polyurethane/polyurea polymers;
(2) an amino cross-linker, which is selected from the class consisting of one or more of urea-formaldehyde, melamine formaldehyde, glycoluril-formaldehyde and benzoguanamine formaldehyde resins which have been at least partially alkylated and at least partially methylolated; and
(3) optionally, an acid catalyst; and
(4) optionally, a solvent; and
(b) heating the applied composition at an elevated temperature and for a time sufficient to cure it;
wherein the polymer (1) is the reaction product of one or more reaction routes selected from the class consisting of (i) self-condensation of a poly-hydroxyalkyl carbamate compound,
(ii) condensation of a poly-hydroxyalkyl carbamate compound with a polyol, and (iii) condensation of a poly-hydroxyalkyl carbamate compound with a polyamine, said poly-hydroxyalkyl carbamate compound having the formula: 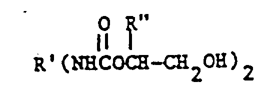 wherein each of R' and R" is the same or different and comprises a suitable organic moiety.

6. The method of Claim 5 including heating the applied composition at a temperature of from about 93° to 204°C (about 200° to about 400°F).

## Patentansprüche

1. Wärmehärtbare Beschichtungszusammensetzung mit:
(a) einem Polymer ausgewählt aus der Klasse bestehend aus einem oder mehreren eine Hydroxygruppe enthaltenden Polyurethan-, eine Hydroxygruppe enthaltenden Polyharnstoff- und eine Hydroxygruppe enthaltenden Polyurethan-/Polyharnstoffpolymeren;
(b) einem Aminovernetzer, der aus der Klasse bestehend aus einem oder mehreren Harnstoff-Formaldehyd-, Melaminformaldehyd-, Glykoluril-Formaldehyd- und Benzoguanaminformaldehydharzen ausgewählt ist, welche wenigstens teilweise alkyliert und wenigstens teilweise methyloliert sind; und
(c) wahlweise einem Säurekatalysator;
wobei das Polymer (a) das Reaktionsprodukt von einem oder mehreren Reaktionswegen ausgewählt aus der Klasse bestehend aus (i) Selbstkondensation einer Poly-hydroxyalkylcarbamatverbindung, (ii) Kondensation einer Poly-hydroxyalkylcarbamatverbindung mit einem Polyol, und (iii) Kondensation einer Poly-hydroxyalkylcarbamatverbindung mit einem Polyamin ist, wobei die Poly-hydroxyalkylcarbamatverbindung folgende Formel aufweist: 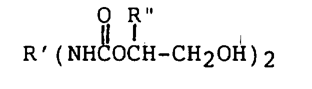 wobei R' und R" jeweils gleich oder verschieden sind und einen geeigneten organischen Anteil enthalten.

2. Zusammensetzung gemäß Anspruch 1, die des weiteren (d) ein Lösemittel enthält, welches mit den Komponenten (a) - (c) kompatibel ist.

3. Zusammensetzung gemäß Anspruch 1, bei der das Polyhydroxyalkylcarbamat Hexamethylen-bis(hydroxypropyl)carbamat ist.

4. Zusammensetzung gemäß Anspruch 1, bei der der Säurekatalysator (c) aus der Klasse bestehend aus einem oder mehreren Lewis- oder Brönsted-Säuren ausgewählt ist.

5. Verfahren zur Bildung einer Beschichtung auf einem Substrat mit
(a) Auftragen einer Beschichtungszusammensetzung auf das Substrat mit:
(1) einem Polymer ausgewählt aus der Klasse bestehend aus einem oder mehreren eine Hydroxygruppe enthaltenden Polyurethan-, eine Hydroxygruppe enthaltenden Polyharnstoff- und eine Hydroxygruppe enthaltenden Polyurethan-/Polyharnstoffpolymeren;
(2) einem Aminovernetzer, der aus der Klasse bestehend aus einem oder mehreren Harnstoff-Formaldehyd-, Melaminformaldehyd-, Glykoluril-Formaldehyd- und Benzoguanaminformaldehydharzen ausgewählt ist, welche wenigstens teilweise alkyliert und wenigstens teilweise methyloliert sind; und
(3) wahlweise einem Säurekatalysator; und
(4) wahlweise einem Lösemittel; und
(b) Erwärmen der aufgetragenen Zusammensetzung bei erhöhter Temperatur und über einen Zeitraum, welcher zu deren Härtung ausreicht;
wobei das Polymer (1) das Reaktionsprodukt von einem oder mehreren Reaktionswegen ausgewählt aus der Klasse bestehend aus (i) Selbstkondensation einer Poly-hydroxyalkylcarbamatverbindung, (ii) Kondensation einer Poly-hydroxyalkylcarbamatverbindung mit einem Polyol, und (iii) Kondensation einer Poly-hydroxyalkylcarbamatverbindung mit einem Polyamin ist, wobei die Poly-hydroxyalkylcarbamatverbindung folgende Formel aufweist: 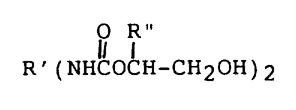 wobei R' und R" jeweils gleich oder verschieden sind und einen geeigneten organischen Anteil enthalten.

6. Verfahren gemäß Anspruch 5, das die Erwärmung der aufgetragenen Zusammensetzung bei einer Temperatur von etwa 93° bis 204°C (etwa 200° bis etwa 400°F) umfaßt.

## Revendications

1. Composition de revêtement thermodurcissable, comprenant:
(a) un polymère choisi dans la classe constituée par un ou plusieurs des polymères polyuréthanes contenant des groupes hydroxy, polyurées contenant des groupes hydroxy et polyuréthanes/polyurées contenant des groupes hydroxy;
(b) un agent de réticulation aminé, qui est choisi dans la classe constituée par une ou plusieurs résines choisies parmi les résines urée-formaldéhyde, mélamine-formaldéhyde, glycolurile-formaldéhyde et benzoguanamine-formaldéhyde, qui ont été au moins partiellement alkylées et au moins partiellement méthylolées; et
(c) le cas échéant, un catalyseur acide; dans laquelle le polymère (a) est le produit de réaction d'une ou plusieurs voies réactionnelles choisies dans la classe constituée par (i) l'autocondensation d'un composé polyhydroxyalkylcarbamate (ii) la condensation d'un composé polyhydroxyalkylcarbamate avec un polyol, et (iii) la condensation d'un composé polyhydroxyalkylcarbamate avec une polyamine, ledit composé polyhydroxyalkylcarbamate répondant à la formule: 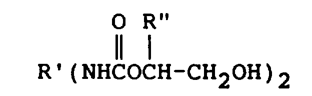 dans laquelle chacun des radicaux R' et R" est identique ou différent, et représente un groupement organique convenable.

2. Composition selon la revendication 1, comprenant en outre, (d) un solvant compatible avec les constituants (a) -(c).

3. Composition selon la revendication 1, dans laquelle le polyhydroxyalkylcarbamate est l'hexaméthylène-bis(hydroxypropyl)carbamate.

4. Composition selon la revendication 1, dans laquelle le catalyseur acide (c) est choisi dans la classe constituée par un ou plusieurs acides de Lewis ou acides de Brönsted.

5. Procédé de formation d'un revêtement sur un substrat, comprenant les étapes qui consistent à:
(a) appliquer sur le substrat une composition de revêtement comprenant:
(1) un polymère choisi dans la classe constituée par un ou plusieurs des polymères polyuréthanes contenant des groupes hydroxy, polyurées contenant des groupes hydroxy et polyuréthanes/polyurées contenant des groupes hydroxy;
(2) un agent de réticulation aminé, qui est choisi dans la classe constituée par une ou plusieurs résines choisies parmi les résines urée-formaldéhyde, mélamine-formaldéhyde, glycolurile-formaldéhyde et benzoguanamine-formaldéhyde, qui ont été au moins partiellement alkylées et au moins partiellement méthylolées; et
(3) le cas échéant, un catalyseur acide; et
(4) le cas échéant, un solvant; et
(b) chauffer la composition appliquée à une température élevée pendant suffisamment longtemps pour la durcir; dans lequel le polymère (1) est le produit de réaction d'une ou plusieurs voies réactionnelles choisies dans la classe constituée par (i) l'autocondensation d'un composé polyhydroxyalkylcarbamate (ii) la condensation d'un composé polyhydroxyalkylcarbamate avec un polyol, et (iii) la condensation d'un composé polyhydroxyalkylcarbamate avec une polyamine, ledit composé polyhydroxyalkylcarbamate répondant à la formule: 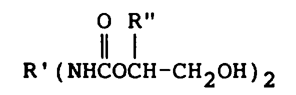 dans laquelle chacun des radicaux R' et R" est identique ou différent, et représente un groupement organique convenable.

6. Procédé selon la revendication 5, comprenant le chauffage de la composition appliquée à une température d'environ 93° à 204°C (environ 200° à environ 400°F).
